# EUROPEAN PATENT SPECIFICATION

(11) **EP 2 948 101 B1**
(45) Date of publication and mention of the grant of the patent: **13.03.2019**
(21) Application number: 14701542.4
(22) Date of filing: 27.01.2014
(51) Int. Cl.: A61F 2/24, A61B 5/107, A61B 5/00

(54) **A MEDICAL DEVICE FOR FACILITATING SELECTION OF AN ANNULOPLASTY IMPLANT**
MEDIZINISCHE VORRICHTUNG ZUR ERLEICHTERUNG DER AUSWAHL EINES ANULOPLASTIEIMPLANTATS
DISPOSITIF MÉDICAL POUR FACILITER LA SÉLECTION D'UN IMPLANT D'ANNULOPLASTIE

(30) Priority: 25.01.2013 EP 13152774; 25.01.2013 US 201361756633 P
(43) Date of publication of application: 02.12.2015
(73) Proprietor: Medtentia International Ltd Oy, 02600 Espoo (FI)
(72) Inventor: KERÄNEN, Olli, S-237 41 Bjärred (SE); VIRTANEN, Jani, 01150 Sipoo (FI); PUGH, Mark, Coolaney Co. Sligo (IE); O'CARROLL, Ger, Co. Sligo (IE); MORAN, Adrian, Co. Sligo (IE)
(86) International application number: PCT/EP2014/051539
(87) International publication number: WO 2014/114794

(56) References cited:
- US-A1- 2001 039 388
- US-A1- 2004 102 722
- US-A1- 2005 010 138
- US-A1- 2006 064 039
- US-A1- 2008 161 825
- US-A1- 2009 276 038
- US-A1- 2010 249 661

## Description

### BACKGROUND OF THE INVENTION

### Field of the invention

This disclosure pertains in general to the field of medical devices. More particularly the disclosure relates to a medical device for facilitating a selection of a size and/or shape of an annuloplasty implant.

### Description of the Prior Art

It is known that a fit of an annuloplasty implant for reshaping cardiac valves is of great importance to ensure greatest possible effect in the repair of the cardiac valve(s). In US2012/197388 a variety of cardiac valve factors are described for choosing a valve body. There is no disclosure of how the factors are acquired and/or used for selection of the annuloplasty implant.

A problem with prior art is how to provide for an easy and reliable way to decide on the shape and/or size of the annuloplasty implant.

A further problem is how to provide for the operator to quickly and with ease visually see which annuloplasty implant the operator should choose. Further problems with prior art include difficulties in positioning such implants.

Thus, there is a need for a medical device acquiring the cardiac valve factors and for use in selection of the annuloplasty implant.

US2009/0276038 discloses a tissue connecting device that comprises an elongate delivery device having a lumen, a proximal end, and a distal end. The distal end is configured to engage tissue and advance said device into tissue.

US2004/0102722 discloses a device and methods for characterizing vulnerable plaque and cancer tissue by measuring changes in tissue elasticity compared to that of normal tissue. The system includes a catheter with an expandable element at a proximal end.

US2010/0249661 discloses a device for measuring an internal dimension of a native cardiac valve annulus includes an elongated support member having a proximal portion and a distal portion.

US2001/0039388 discloses an apparatus for measuring length in a blood vessel that includes an inner barrel slideably fitting within an outer barrel.

US2005/0010138 provides a lumen measuring device and method that allows the user to calculate the exact length and diameter of a suitable interventional prosthesis as well as the height and length of stenosis during the same exploratory procedure.

US2008/0161825 a measuring device for measuring tunnel defects in tissue is disclosed. The measuring device can size the defect to aid future deployment of a tissue distension device.

US2006/0064039 discloses a measurement device used to measure a size of a lumen and related methods of use are disclosed.

### SUMMARY OF THE INVENTION

The invention relates to a medical device and is defined by the appended claims.

Accordingly, examples of the present disclosure preferably seek to mitigate, alleviate or eliminate one or more deficiencies, disadvantages or issues in the art, such as the above-identified, singly or in any combination by providing a medical device and a method for use thereof that facilitates a selection of a size and/or shape of an annuloplasty implant, according to the appended patent claims.

According to aspects of the invention, a medical device for facilitating selection of a shape and/or size of an annuloplasty implant for a patient is provided.

According to a first aspect of the invention, a medical device is provided, wherein the medical device comprises a catheter with a proximal end and a distal end. The medical device further comprises an extension member at least partly arranged inside the catheter with an operator end and a measurement end and wherein the measurement end of the extension member is extendable relative from the distal end of the catheter for apposition with at least one commissure of a cardiac valve, such as a mitral valve of the patient. The measurement end of the extension member comprises means for guiding the annuloplasty implant at at least one commissure.

According to another example, a method is provided, wherein the method is providing for facilitating selection of a shape and/or size of an annuloplasty implant. The method comprises providing a medical device such as the medical device for facilitating the selection of a shape and/or size of an annuloplasty implant as described above. The method further comprises positioning, preferably minimally invasively, a distal end of the catheter of the medical device at a cardiac valve of a patient. The method further comprises extending a measurement end of an extension member relative from a distal end of the catheter, bringing the measurement end in apposition with at least one commissure of the cardiac valve, such as a mitral valve of said patient. The method comprises further guiding the annuloplasty implant to at least one commissure by use of the extension member comprising means for guiding the annuloplasty implant.
Further examples of the disclosure are defined in the dependent claims, wherein features for the second and subsequent aspects of the disclosure are as for the first aspect mutatis mutandis.

Some examples of the disclosure provide for easy positioning of an implant at a valve.

Some examples of the disclosure provide for efficient stabilizing of the valve before positioning an implant.

Some examples of the disclosure provide for atraumatic guiding and positioning of an implant.

Some examples of the disclosure provide for an easy and reliable way to decide on the shape and/or size of the annuloplasty implant.

Some examples of the disclosure also provide for the operator to quickly and with ease visually see which annuloplasty implant the operator should choose.

Some examples of the disclosure also provide for easy use and movement of the extension member and catheter.

Some examples of the disclosure also provide for easy use and movement of the extension member and catheter independently of each other.

Some examples of the disclosure also provide for an extension member comprising a suitable material compatible with and for use in a catheter and in a heart.

Some examples of the disclosure also provide for a distance between two commissures to be measured immediately and relatively short amount of time.

Some examples of the disclosure also provide for an easier to control extension member due to shared alignment of two separable sections with the direction of a catheter.

Some examples of the disclosure also provide for an extension member that are easier to apposition with the two commissures due to the extension members having two separable extensions, or sections, being synchronized when extended.

Some examples of the disclosure also provide for easier manufacturing of the extension member comprising two separable sections since the extension member and the two separable extensions are made in one piece.

Some examples of the disclosure also provide for an increased breaking resistance and/or improved rotational force of an extension member and two separable sections.

Some examples of the disclosure also provide for an extension member and two separable sections to be manufactured from different material and thus have different material properties with respect to bending, rotation and/or biocompatibility.

Some examples of the disclosure also provide for a more reliable indication of when an extension member is at apposition or in contact to or with at least one commissure.

Some examples of the disclosure also provide for attaching at least one anchor at the at least one commissure to so that an annuloplasty implant can be anchored.

Some examples of the disclosure also provide for an annuloplasty implant to be anchored at different locations from different entering points at the commissure which provides for stabilizing the annuloplasty implant at these suitable locations.

Some examples of the disclosure also provide for an annuloplasty implant, which preferably has the shape of a helix ring, to be rotated into place at the cardiac valve by use of anchors.

Some examples of the disclosure also provide for a rotation of an extension member relative to a catheter.

Some examples of the disclosure also provide for a sliding of an extension member inside a catheter.

Some examples of the disclosure also provide for a synchronised movement of a catheter and an extension member.

Some examples of the disclosure also provide for a synchronised movement of a catheter and an extension member where the extension member and the catheter is engaged so that when movement of the catheter is performed the extension member is moved in the same way as the catheter.

Some examples of the disclosure also provide for an extended length of an extension member from a substantially centre position in a cardiac valve to an at least one commissure resulting in a measure on a size and/or shape of an annuloplasty implant.

Some examples of the disclosure also provide for an extension member comprising two separable sections separable towards two commissures to result in a measure of a width between the two commissures which is very accurate and quickly acquired.

Some examples of the disclosure also provide for an extension of the extension member from a catheter to be performed out from the proximal end of the catheter.

Some examples of the disclosure also provide for an extension of the extension member from a catheter to be performed out through the sidewall of the catheter at a proximal end of the catheter.

Some examples of the disclosure also provide for an indication of when at least one commissure has been found to be performed more reliable by use of a maneuver force detection unit.

Some examples of the disclosure also provide for an indication of an apposition or contact to an at least one commissure by comparing a measured applied maneuver force with a predefined commissure value for triggering the indication.

Some examples of the disclosure also provide for an operator to locate at least one commissure and attach anchors for an annulopasty implant at the at least one commissure in one go and with the same device, saving time compared to needed to use a second instrument for attaching anchors.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which
Fig. 1 is a cross-sectional view of an example of a medical device for facilitating a selection of a size and/or shape of an annuloplasty implant positioned at cardiac valves;
Figs. 2a-b is a side view of another example of a medical device for facilitating a selection of a size and/or shape of an annuloplasty implant positioned at cardiac valves;
Fig. 3 is a cross-sectional view of another example of a medical device for facilitating a selection of a size and/or shape of an annuloplasty implant positioned at cardiac valves;
Figs. 4a-c is a side view of another example of a medical device for facilitating a selection of a size and/or shape of an annuloplasty implant positioned at cardiac valves;
Fig. 5 is a cross-sectional view of another example of a medical device for facilitating a selection of a size and/or shape of an annuloplasty implant positioned at cardiac valves;
Fig. 6 is a flowchart of a method for facilitating a selection of a size and/or shape of an annuloplasty implant;
Fig. 7 is a side view of another example of a medical device for facilitating a selection of a size and/or shape of an annuloplasty implant comprising a means for limiting leaflet movement; and
Figs. 8a-b, are views of another example of the device in Fig. 7.

### DESCRIPTION OF THE PREFERRED EMBODIMENTS

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on an example of the present disclosure applicable to a medical device and in particular to a medical device for facilitating selection of a shape and/or size of an annuloplasty implant.

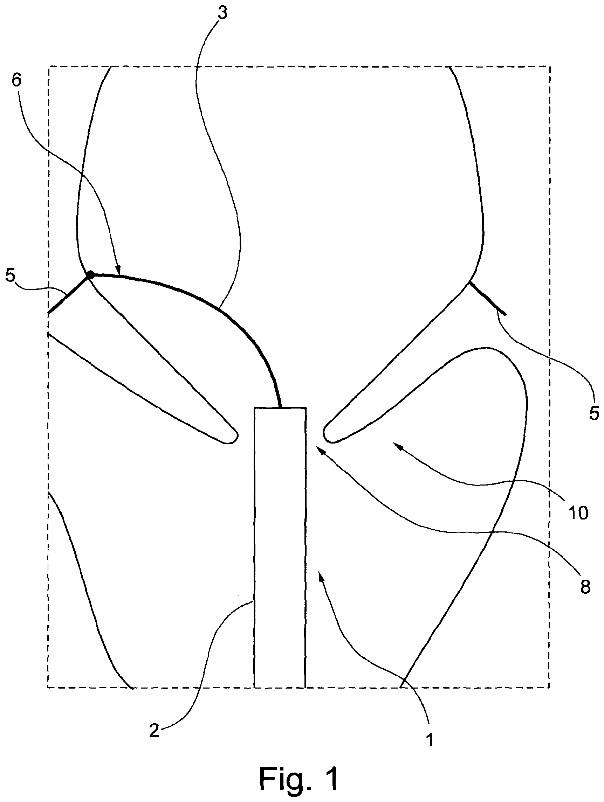

In an example of the disclosure according to Fig. 1 a medical device 1 for facilitating selection of a shape and/or size of an annuloplasty implant for a patient. The medical device 1 comprises a catheter 2 with a proximal end and a distal end 8. The medical device 1 further comprises an extension member 3 at least partly arranged inside the catheter 2 with an operator end and a measurement end 9 and wherein the measurement end 9 of the extension member 3 is extendable relative from the distal end of the catheter 2 for apposition with at least one commissure 5 of a cardiac valve 10, such as a mitral valve of the patient and wherein a measure related to the selection of the annuloplasty implant shape and/or size is based on at least an extended length of the measurement end of the extension member 3 from the distal end of the catheter 2, positioned at the cardiac valve, to the at least one commissure 5. By use of the medical device 1 providing the measure related to the selection of the shape and/or size of the annuloplasty implant an operator of the medical device 1 is facilitated to in an easy and reliable way decide on the shape and/or size of the annuloplasty implant.

Catheter 2 used herein this disclosure is of well known types and wherein the catheter 2 is capable of comprising at least an extension member 3 according to this disclosure. Additionally the catheter 2 is capable of being rotated and/or otherwise steered into position at the cardiac valve from a desired position in or outside the body by the operator.

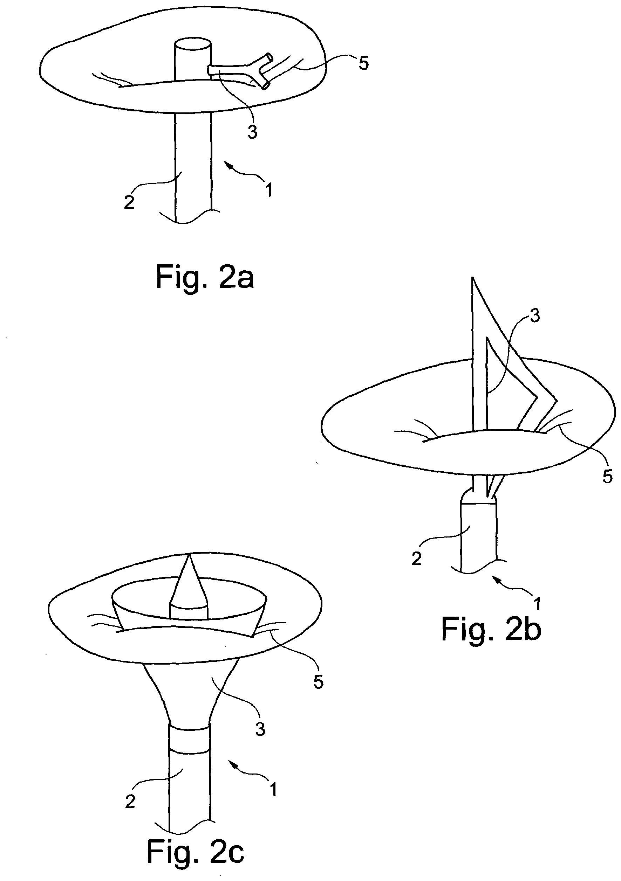

In an example the extension member 3 is a rod or alternatively a pole and/or another long thin member with a cylindrical, circular, squared or rectangular base, capable of being arranged in the catheter 2. In an example illustrated in Fig. 2a the extension member 3 is a rod extended perpendicular from the catheter 2 outwards towards the commissure. In another example illustrated in Fig. 2b the extension member is of a semi-circular shape such as a leaf shaped and where the semi-circular shape is directed towards the at least one commissure and has a spring action for apposition to at least one commissure. Another example of the extension member is illustrated in Fig. 2c where the extension member has an oval cone shape for apposition to at least one commissure. The oval cone shape is in example formed of at least one extending sheet. In another example the oval cone shape is formed from several braided, extending or interwoven shape members.

In another example the extension member 3 is rotationally arranged in the catheter 2 for apposition with the at least one commissure 5. In another example the extension member 3 is slidably arranged in the catheter 2. These arrangements allow for easy use and movement of the extension member 3 and catheter 2. Alternatively, the arrangement allows for easy use and movement independently of each other.

The extension member 3 is made of a suitable material compatible with and for use in a catheter 2 and in a heart, such as of titanium, nitinol, polymer, carbon fiber, textiles, all in solid forms or in braided or sandwich structure forms, etc. The extension member 3 has a length that is at least as long as the catheter 2 and a distance from the catheter 2 to the at least one commissure 5. The extension member 3 is preferably long enough to be operated at the operator end by the proximal end of the catheter 2 and still extendable at the measurement end at the distal end of the catheter 2, i.e. the extension member 3 extends out of and from the catheter 2 at both ends of the catheter 2 when used by the operator.

In another example of the extension member 3 the extension member 3 has a length wherein the measurement end of the extension member 3 only extends out and from the distal end of the catheter 2 and the operator end of the extension member 3 is arranged at level with the proximal end of the catheter 2, i.e. the extension member 3 only extends from the catheter 2 at the distal end of the catheter 2 when used by the operator. By using the manoeuvrable extension member 3 the operator measures a distance from the catheter 2 at the cardiac valve to the at least one commissure 5 and bases the size and/or shape of the annuloplasty implant on the distance.

In one example the measure related to the annuloplasty implant's shape and/or size is indicated at the operator end of the extension member 3. By having the operator end of the extension member 3 indicating the measure related to the size and/or shape of the annuloplasty implant, the operator can quickly and with ease visually see which annuloplasty implant the operator should choose.

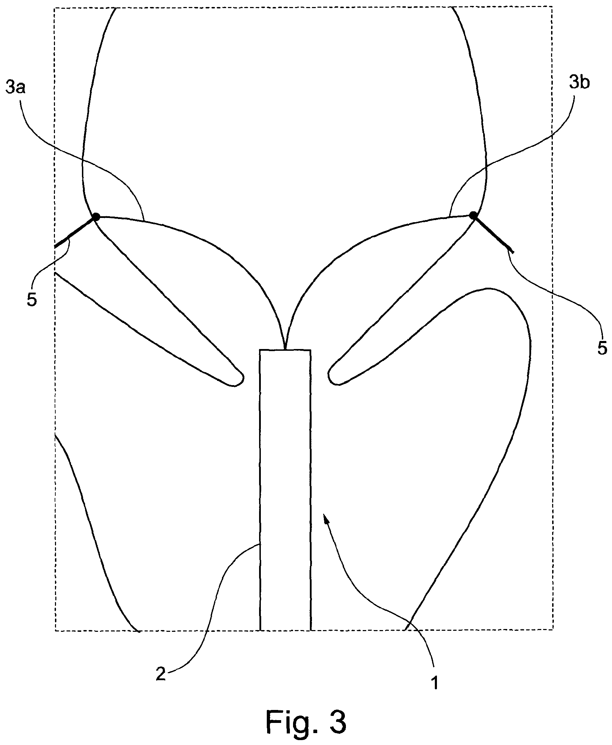

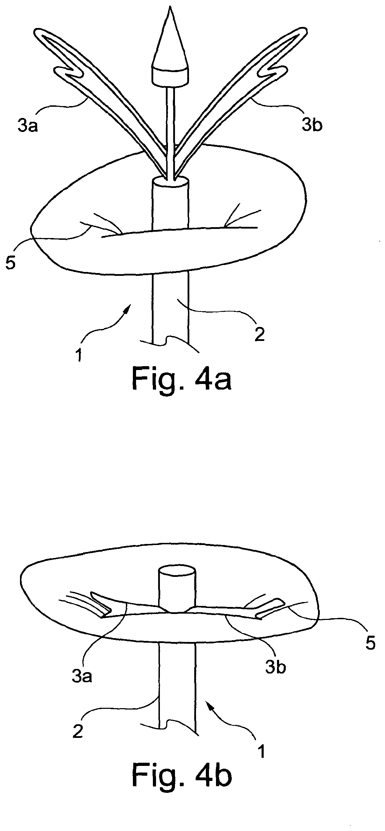

As illustrated in Figs. 3, 4a and 4b, another example of the measurement end of the extension member 3 comprises two sections 3a, 3b, separable towards each of the mitral valve's commissures 5. By using two sections 3a, 3b, that are separable towards two commissures 5 at the mitral valve a distance between the two commissures 5 is measured immediately and faster than when using the extension member 3 without the two separable sections 3a, 3b.

In other example the two separable sections 3a, 3b, are upon extension from the catheter 2 aligned in a plane extending along a direction of the proximal end of the catheter 2. By having the two separable sections 3a, 3b, aligned and extended in the plane parallel to the direction of the catheter 2 the two sections 3a, 3b, will be easier to control due to their shared alignment with the direction of the catheter 2. This can be illustrated in Fig. 4b where the separable sections 3a, 3b, of the extension member 3 are separated perpendicular to the catheter 2.

Further illustrated in Fig. 4b, in yet another example the two separable sections 3a, 3b, separate with an opposite inclined separation angle. By having the two sections 3a, 3b, separate with opposite inclined angle of separation the two separable sections 3a, 3b, extend the same distance outwards towards the commissures 5 and thus are easier to apposition with the two commissures 5 due to their synchronised extension.

The two separable sections 3a, 3b, are in one example an integral continuation of the extension member 3. By having the two separable sections 3a, 3b, being the integral continuation of the extension member 3 the two separable sections 3a, 3b, better responds to manoeuvres, such as rotation and/or extension of the extension member 3 performed by the operator. Additionally, a requirement for manufacturing of the extension member 3 is greatly reduced since the extension member 3 and the two separable extensions are made in one piece. In one example the two separable sections 3a, 3b, and the extension member's 3 mechanical aspects such as increased breaking resistance and/or improved rotational force, are greatly improved because the extension member 3 and the two separable sections 3a, 3b, are sized and/or shaped dependent on each other.

Alternatively, the two separable sections 3a, 3b, are joined to the measurement end of the extension member 3. By allowing the two separable sections 3a, 3b, to be joined at the measurement end of the extension member 3 they may be manufactured from a different material than the extension member 3 and thus have other material properties with respect to bending, rotation and/or biocompatibility.

In another example illustrated in Fig. 4a the extension member 3 comprises two separable sections 3a, 3b, which further comprises a c-shaped or claw shaped end. This claw shaped end is large enough to encompass an edge of a valve leaflet when aligned at the at least one commissure so that the extension member is further secured at the at least one commissure.

In one example the medical device 1 further comprises a force detection unit connected to the extension member 3 for detection of a manoeuvre force applied to the extension member 3. By using the force detection unit for detecting the manoeuvre force applied to the extension member 3 it is possible to get a further more reliable indication of when the extension member 3 is at apposition or in contact to or with at least one commissure 5.

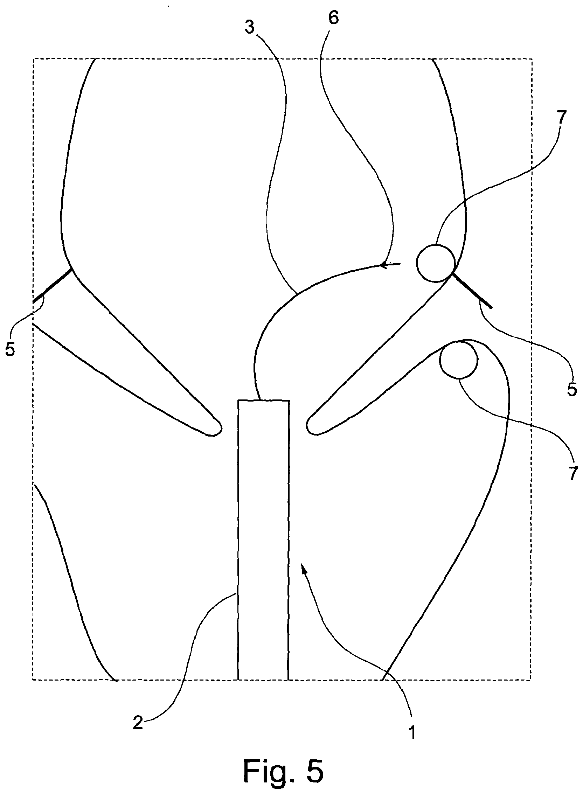

In a further example of the extension member 3, as illustrated in Fig. 5, the measurement end of the extension member 3 comprises anchoring means 6 for attaching anchors 7 at at least one commissure 5 for the annuloplasty implant. Alternatively, one anchor 7 is attached at one commissure 5. By having the extension member 3 comprising anchoring means 6 for attaching anchors 7 for the annuloplasty implant it is possible to detect the location of the at least one commissure 5 and following the localization attach anchors 7 at the commissure 5 so that the annuloplasty implant can be anchored. This allows for fast deployment of the annuloplasty implant after the at least one commissure 5 is found and the size and/or shape of the annuloplasty implant has been chosen. In an example the anchoring means 6 is a claw or similar that allows for gripping the anchors.

In one example, the anchors 7 comprise at least one guiding unit or ring, as also illustrated in Fig. 5. By using at least one guiding unit or rings as anchors 7 the annuloplasty implant, which preferably has the shape of a helix ring, is rotated into place at the cardiac valve by use of the anchors 7. For example, when using rings as anchors 7 the annuloplasty implant is inserted through and slides in the rings securing the annuloplasty implant at the commissures 5. In an example the anchors 7 are arranged in the atrium and catch and guides an upper part of the helix ring. In another example the anchors 7 are arranged in the ventricle and catch and guides a lower part of the helix ring. In yet another example the anchors 7 are arranged in both the atrium and the ventricle, which is illustrated in Fig. 5, and catch both parts of the helix ring and part of the annulus. This allows for the helix ring to be anchored in different ways from different entering points at the commissure and provides for stabilizing the helix ring at suitable locations.

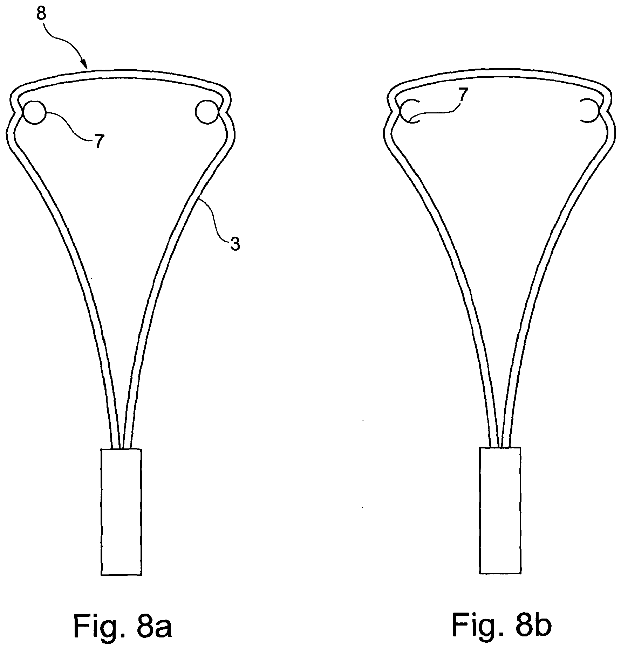

As discussed above, in one example the anchoring means 6 comprises anchors 7 that are used as guides, i.e. guiding means, for the annuloplasty implant at the at least one commissure. In another example the anchors 7 are used alternatively and/or in addition, as means for guiding the annuloplasty implant at the at least one commissure before the anchors 7 may be attached at the at least one commissure. This allows the user to both measure the correct size of the annuloplasty implant and guide the annuloplasty implant into place in an easy way without removing the medical device 1 when placed at the at least one commissure 5 and at the same time avoid attaching the anchors 7 at the at least one commissure, thus reducing the time for deploying the annuloplasty implant in the patient. In this example, the means for guiding is may be generally open or c-shaped which allows the annuloplasy implant to be guided into place in the heart without attaching the means for guiding at the at least one commissure and which allows for removal of the means for guiding, after the annuloplasty implant is implanted in the patient, through the opening of the c-shape. Fig. 8b shows the extension member comprising guiding means 7 that are generally open or C-shaped for guiding an implant into place. Other shapes that can be used are substantially loop-shaped, triangle-shaped, ring-shaped, such as shown in Fig. 8a, or any other suitable shape that allows for guiding the annuloplasty implant into place and/or allows for removing the means for guiding when the annuloplasty implant is implanted in the heart. As shown in Figs. 8a-b the extension member may have guiding means 7 at each the two lateral parts of the extension member that are to be placed at the commissures.

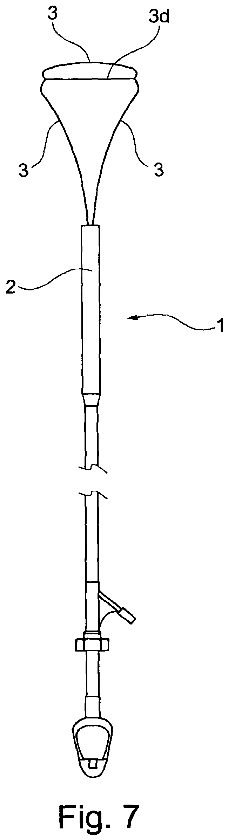

In a further example of the extension member 3, as illustrated in Fig. 7, the measurement end of the extension member 3 is shaped and/or formed as one coherent member 3. The extension member 3 may thus be formed as a continuous single or one-piece loop, i.e. a closed design. By using the extension member 3 formed from one piece closed design the member 3 is much more stable in its construction and easier to manoeuvre in the heart. Further, the continuous loop provides for particularly efficient stabilization of the anatomy and improving the precision by which the implant can be placed at the valve. Further, the continuous loop minimizes undesired interference with the chordae in the heart that would otherwise be the risk when having projections, edges, kinks etc. The extension member may comprise a continuous loop having a distal portion 8 being curved outwardly in a direction from the distal end of the catheter, see Fig. 8a. Such curved shape further reduces the risk of damaging any chordae due the smooth shape. In the example in Fig. 8a, the distal portion 8 bridges the two guiding means 7 on the extension member. This provides for an atraumatic extension member that effectively stabilizes the valve, while at the same time providing guiding means for the implant. The principle of use and mode of use is the same as for the other examples of extension members 3 described in this application. Hence, the measurement, expansion, material and so on are the same and operates in the same way.

In another example, as also illustrated in figure 7, the extension member 3 comprises a leaflet limiter 3d.The leaflet limiter 3d is not limited to be used only with the coherent extension member 3 but the other types of extension members 3 disclosed in this application may also have the leaflet limiter 3d. The leaflet limiter 3d limits abnormal movement, such as prolapse, of the leaflets into the atrium. Such abnormal movement may arise if a chordae, or several chordae, that usually limits the movement of the leaflet is completely destroyed and the leaflet may thus freely move in the left atrium and/or left chamber. The leaflet limiter 3d is made of a material that expands with the extension member 3, and it may be made of the same material as the extension member 3. The leaflet limiter 3d may also be such that it can be bent, twisted or otherwise collapsed into the catheter 2 and then assume a desired shape when released from the catheter 2. Alternatively, the leaflet limiter 3d is expanded by a spring back motion and/or force when exited from the catheter 2 with the extension member 3d. The example of the leaflet limiter 3d shown in fig. 7 is a crossbar that extends between two anchoring points of the extension member 3 and is projected laterally from an intersecting plane of the anchoring points of the extension member 3. The leaflet limiter 3d may be of one piece or be made up of several pieces and/or have a number of different shapes and/or have various placements. One example of a shape that limits but not damage the leaflet(s) when hindering the movement into the atrium would be to have a simple straight projection outwards towards the leaflets from the extension member 3 with a blunt end, which can limit the movement but not damage the leaflet(s) when hindering the movement into the atrium. Preferably, the extension member 3 has two leaflet limiters 3d, one on each side of the extension member 3 for each leaflet when the extension member 3 is arranged at the commissures. But, there could also be only one leaflet limiter 3d. This could be the case if it is known that one leaflet is already damaged and moving freely when starting the procedure of measuring and/or deciding the size of the annuloplasty implant.

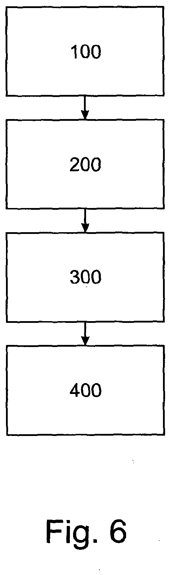

In an example according to the disclosure and as illustrated in Fig. 6 is a method for facilitating selection of a shape and/or size of an annuloplasty implant. The method comprises providing 100 a medical device 1 such as the medical device 1 for facilitating the selection of a shape and/or size of an annuloplasty implant as described above. The method further comprises positioning 200, preferably minimally invasively, a distal end of the catheter 2 of the medical device 1 at a cardiac valve of a patient. The method further comprises extending 300 a measurement end of an extension member 3 relative from a distal end of the catheter 2, bringing the measurement end in apposition with at least one commissure 5 of the cardiac valve, such as a mitral valve of said patient. The method also comprises basing 400 the annuloplasty implant's shape and/or size on at least an extended length of the extension member 3 relative from the distal end of the catheter 2 to the at least one commissure 5. By using the medical device 1 for facilitating the selection of the shape and/or size of the annuloplasty implant comprising the catheter 2 and the extension member 3 it is possible to base the size/and or shape of the annuloplasty implant on the extension of the extension member 3 relative from the catheter 2.

In one example, the catheter 2 is positioned in a substantially centre position at the cardiac valves. Following the extension member 3 is extended from the distal end of the catheter 2 by an operator pushing the extension member 3 from the proximal end of the catheter 2 through the catheter 2 and out at the distal end of the catheter 2. The measurement end of the extended extension member 3 is positioned at, appositioned, or in contact with the commissure 5.

The positioning of the extension member 3 is performed in a number of way such as by rotating the extension member 3 relative to the catheter 2, sliding the extension member 3 inside the catheter 2, by synchronised movement of the catheter 2 and the extension member 3 and/or by synchronised movement of the catheter 2 and the extension member 3 where the extension member 3 and the catheter 2 is engaged so that when movement of the catheter 2 is performed the extension member 3 is moved in the same way as the catheter 2.

The extended length of the extension member 3 from the substantially centre position to the commissure 5 gives the operator a measure on the size and/or shape of the annuloplasty implant. The extended length is in one example used as basis for the radius of the annuloplasty implant. In another example an assumption that the cardiac valve is symmetrical together with the extended length of the extension member 3 is used as basis for the width of the annuloplasty device.

In another example of the method for facilitating selection of a shape and/or size of an annuloplasty implant the basing of the annuloplasty implant's shape and/or size is based on a measured valve width between two commissures 5 of the cardiac valve by the extension of the measurement end of the extension member 3 relative from the catheter 2 to the two commissures 5. Basing the selection of the annuloplasty implant on the distance between the two commissures 5 gives a better fit of the annuloplasty implant than when only using one commissure 5. In one example the width between the two commissures 5 are measured by sweeping the extension member 3 from one commissure 5 to the other commissure 5.

In another example the width is obtained between the two commissures 5 by arranging of two separable sections 3a, 3b, of the extension member 3 separable towards the commissures 5. The use of the extension member 3 comprising two separable sections 3a, 3b, separable towards the commissures 5 results in the width between the commissures 5 being measured more accurately and faster than any presently known method. When obtaining the width between the commissures 5 by use of the extension member 3 comprising two separable sections 3a, 3b, the operator positions the catheter 2 at the cardiac valve and extends the extension member 3. The two separable sections 3a, 3b, separate outwards towards the commissures 5 when they passes the distal end of the catheter 2 by the operator pushing the extension member 3 through the catheter 2 from the proximal end of the catheter 2. Depending on the pushed distance of the extension member 3 i.e. extended distance of the extension member 3 and the two separable sections 3a, 3b, the width of the commissures 5 is known. The separation of the two separable sections 3a, 3b, is preferably at a predefined angle and/or settles at the predefined angle when measuring the width between the commissures 5. The extension of the extension member 3 from the catheter 2 may be performed in several ways such as, out from the proximal end of the catheter 2 and/or out through the sidewall of the catheter 2 at the proximal end.

In one example the method further comprises measuring an applied manoeuvre force on the extension member 3 while manoeuvring the extension member 3 to apposition the measurement end with the at least one commissure 5 and, indicating when the measurement end is apposition with the at least one commissure 5 based on the measured applied manoeuvre force. By measuring the applied manoeuvre force on the extension member 3 applied by the operator the indication of when at least one commissure 5 has been found is performed more reliable than by use of tactile indication through the extension member 3. The measurement of the applied manoeuvre force may e.g. be measured by a force detection unit.

In one example if the force detection unit is used, the force detection unit bases the indication of the apposition to the at least one commissure 5 by comparing the measured applied manoeuvre force with a predefined commissure 5 value for triggering the indication of the apposition of the measurement end with the at least one commissure 5.

In another example of the method for facilitating selection of a shape and/or size of an annuloplasty implant an indication is based on a measured force for stretching the extension member 3 between two commissures 5. By measuring the force needed to extend and/or stretch the extension member 3 outwards towards the two commissures 5 it is possible to detect when the two commissures 5 have been found since the two commissures 5 have a difference in flexibility compared to other tissue in the atrium.

In yet another example the method comprises anchoring at least one anchor at at least one commissure 5 by use of the extension member 3 comprising anchoring means. By using at least one anchor at at least one commissure 5 by using the extension member 3 the operator can attach anchors for the annulopasty device in one go and with the same device, saving time compared to needed to use a second instrument for attaching anchors. In one example the anchoring means 6 comprises anchors 7, or guiding means, that are used as guides for the annuloplasty implant at the at least one commissure. In another example the anchors 7 are used alternatively and/or in addition, as means for guiding the annuloplasty implant at the at least one commissure before the anchors 7 are attached at the at least one commissure. This allows the user to both measure the correct size of the annuloplasty implant and guide the annuloplasty implant into place in an easy way without removing the medical device 1 when placed at the at least one commissure 5 and at the same time avoid attaching the anchors 7 at the at least one commissure, thus reducing the time for deploying the annuloplasty implant in the patient. In this example, the means for guiding is preferably c-shaped which allows the annuloplasy implant to be guided into place in the heart without attaching the means for guiding at the at least one commissure and allows for removal of the means for guiding after the annuloplasty implant is implanted in the patient through the opening of the c-shape. Other shapes that can be used are loop-shaped, ring-shaped or any other suitable shape that allows for guiding the annuloplasty implant into place and/or allows for removing the means for guiding when the annuloplasty implant is implanted in the heart.

The present disclosure has been described above with reference to specific examples. However, other examples than the above described are equally possible within the scope of the disclosure. Different method steps than those described above, performing the method by hardware or software, may be provided within the scope of the disclosure. The different features and steps of the disclosure may be combined in other combinations than those described. The scope of the disclosure is only limited by the appended patent claims.

## Claims

1. A medical device (1) for facilitating selection of a shape and/or size of an annuloplasty implant for a patient, comprising:
a catheter (2) with a proximal end and a distal end (8),
an extension member (3) at least partly arranged inside the catheter (2) with an operator end and a measurement end (9),
wherein the measurement end of the extension member is extendable relative from the distal end of the catheter for apposition with at least one commissure of a cardiac valve, such as a mitral valve of the patient,
wherein a measure related to the selection of the annuloplasty implant's shape and/or size is based on at least an extended length of the measurement end (9) of the extension member (3) from the distal end of (8) the catheter (2), positioned at the cardiac valve, to the at least one commissure, **characterized in that**
the measurement end (9) of the extension member (3) comprises means for guiding the annuloplasty implant at at least one commissure.

2. The medical device according to claim 1, wherein the means for guiding is loop-shaped or c-shaped.

3. The medical device according to any of claims 1-3, wherein the measure related to the annuloplasty implant's shape and/or size is indicated at the operator end of the extension member.

4. The medical device according to any of claims 1-4, wherein the measurement end of the extension member (3) comprises two sections (3a, 3b) separable towards each of the mitral valve's commissures.

5. The medical device according to claim 5, wherein the two separable sections (3a, 3b) are upon extension from the catheter (2) aligned in a plane extending along a direction of the proximal end of the catheter.

6. The medical device according to claims 5 or 6, wherein the two separable sections (3a, 3b) separate with an opposite inclined separation angle.

7. The medical device according to any of claims 5-7, wherein the two separable sections (3a, 3b) are an integral continuation of the extension member (3).

8. The medical device according to any of claims 5-7, wherein the two separable sections (3a, 3b) are joined to the measurement end (9) of the extension member (3).

9. The medical device according any of the preceding claims, further comprising a force detection unit connected to the extension member (3) for detection of a manoeuvre force applied to the extension member (3).

10. The medical device according any of the preceding claims, wherein the extension member (3) is rotationally arranged in the catheter (2) for apposition with the at least one commissure.

11. The medical device according any of the preceding claims, wherein the measurement end (9) of the extension member (3) comprises anchoring means (6) for attaching anchors at at least one commissure for the annuloplasty implant.

12. The medical device according to claim 12, wherein the anchors comprise at least one guiding unit or ring.

13. The medical device according any of the preceding claims, further comprising a leaflet limiter (3d) arranged at the extension member (3).

14. The medical device according to claim 14, wherein the leaflet limiter (3d) is self-expandable from a delivery mode to an expanded mode.

15. The medical device according to any of claim 1-4 or 10-15, wherein the extension member (3) is formed as a continuous single or one-piece loop.

16. The medical device according to claim 16, wherein the extension member (3) comprises a continuous loop having a distal portion being curved outwardly in a direction from the distal end (8) of the catheter (2).

## Patentansprüche

1. Medizinische Vorrichtung (1) zur Erleichterung der Auswahl einer Form und/oder Größe eines Annuloplastieimplantats für einen Patienten, umfassend:
einen Katheter (2) mit einem proximalen Ende und einem distalen Ende (8),
ein zumindest teilweise innerhalb des Katheters (2) angeordnetes Verlängerungselement (3) mit einem Bedienerende und einem Messende (9),
wobei das Messende des Verlängerungselements zur Apposition mit zumindest einer Kommissur einer Herzklappe, wie beispielsweise einer Mitralklappe des Patienten, relativ vom distalen Ende des Katheters ausziehbar ist, wobei ein Maß in Zusammenhang mit der Auswahl der Form und/oder Größe des Annuloplastieimplantats zumindest auf einer ausgezogenen Länge des Messendes (9) des Verlängerungselements (3) vom distalen Ende (8) des Katheters (2), der an der Herzklappe positioniert ist, bis zur zumindest einen Kommissur beruht, **dadurch gekennzeichnet, dass**
das Messende (9) des Verlängerungselements (3) Mittel zum Führen des Annuloplastieimplantats an zumindest einer Kommissur umfasst.

2. Medizinische Vorrichtung nach Anspruch 1, wobei das Mittel zum Führen schleifenförmig oder C-förmig ist.

3. Medizinische Vorrichtung nach einem der Ansprüche 1-3, wobei das Maß in Zusammenhang mit der Form und/oder Größe des Annuloplastieimplantats am Bedienerende des Verlängerungselements angezeigt wird.

4. Medizinische Vorrichtung nach einem der Ansprüche 1-4, wobei das Messende des Verlängerungselements (3) zwei Abschnitte (3a, 3b) umfasst, die zu jeder der Kommissuren der Mitralklappe trennbar sind.

5. Medizinische Vorrichtung nach Anspruch 5, wobei die zwei trennbaren Abschnitte (3a, 3b) nach Ausziehen aus dem Katheter (2) in einer Ebene ausgerichtet sind, die sich entlang einer Richtung des proximalen Endes des Katheters erstreckt.

6. Medizinische Vorrichtung nach den Ansprüchen 5 oder 6, wobei die zwei trennbaren Abschnitte (3a, 3b) sich mit einem entgegengesetzten geneigten Trennwinkel trennen.

7. Medizinische Vorrichtung nach einem der Ansprüche 5-7, wobei die zwei trennbaren Abschnitte (3a, 3b) eine einstückige Fortsetzung des Verlängerungselements (3) sind.

8. Medizinische Vorrichtung nach einem der Ansprüche 5-7, wobei die zwei trennbaren Abschnitte (3a, 3b) mit dem Messende (9) des Verlängerungselements (3) verbunden sind.

9. Medizinische Vorrichtung nach einem der vorhergehenden Ansprüche, ferner umfassend eine Kraftnachweiseinheit, die mit dem Verlängerungselement (3) verbunden ist, um eine auf das Verlängerungselement (3) aufgebrachte Manöverkraft nachzuweisen.

10. Medizinische Vorrichtung nach einem der vorhergehenden Ansprüche, wobei das Verlängerungselement (3) zur Apposition mit der zumindest einen Kommissur drehbar im Katheter (2) angeordnet ist.

11. Medizinische Vorrichtung nach einem der vorhergehenden Ansprüche, wobei das Messende (9) des Verlängerungselements (3) Verankerungsmittel (6) zur Befestigung von Ankern an zumindest einer Kommissur für das Annuloplastieimplantat umfasst.

12. Medizinische Vorrichtung nach Anspruch 12, wobei die Anker zumindest eine Führungseinheit oder einen Führungsring umfassen.

13. Medizinische Vorrichtung nach einem der vorhergehenden Ansprüche, ferner umfassend einen Segelbegrenzer (3d), der am Verlängerungselement (3) angeordnet ist.

14. Medizinische Vorrichtung nach Anspruch 14, wobei der Segelbegrenzer (3d) aus einem Freisetzungsmodus in einen expandierten Modus selbst expandierbar ist.

15. Medizinische Vorrichtung nach einem der Ansprüche 1-4 oder 10-15, wobei das Verlängerungselement (3) als kontinuierliche einzelne oder einstückige Schleife ausgebildet ist.

16. Medizinische Vorrichtung nach Anspruch 16, wobei das Verlängerungselement (3) eine kontinuierliche Schleife mit einem distalen Abschnitt umfasst, der in einer Richtung vom distalen Ende (8) des Katheters (2) nach oben gebogen ist.

## Revendications

1. Dispositif médical (1) pour faciliter la sélection d'une forme et/ou d'une taille d'un implant d'annuloplastie pour un patient, comprenant :
un cathéter (2) avec une extrémité proximale et une extrémité distale (8),
un élément d'extension (3) au moins partiellement agencé à l'intérieur du cathéter (2) avec une extrémité d'opérateur et une extrémité de mesure (9),
dans lequel l'extrémité de mesure de l'élément d'extension est extensible par rapport à l'extrémité distale du cathéter pour apposition à au moins une commissure d'une valvule cardiaque, telle qu'une valvule mitrale, du patient,
dans lequel une mesure concernant la sélection de la forme et/ou de la taille de l'implant d'annuloplastie est basée sur au moins une longueur étendue de l'extrémité de mesure (9) de l'élément d'extension (3) à partir de l'extrémité distale (8) du cathéter (2), positionnée à la valvule cardiaque, jusqu'à ladite au moins une commissure,
**caractérisé en ce que** l'extrémité de mesure (9) de l'élément d'extension (3) comprend des moyens pour guider l'implant d'annuloplastie au niveau d'au moins une commissure.

2. Dispositif médical selon la revendication 1, dans lequel les moyens de guidage sont en forme de boucle ou en forme de C.

3. Dispositif médical selon l'une quelconque des revendications 1 à 3, dans lequel la mesure concernant la forme et/ou la taille de l'implant d'annuloplastie est indiquée à l'extrémité de l'opérateur de l'élément d'extension.

4. Dispositif médical selon l'une quelconque des revendications 1 à 4, dans lequel l'extrémité de mesure de l'élément d'extension (3) comprend deux sections (3a, 3b) séparables en direction de chacune des commissures de la valvule mitrale.

5. Dispositif médical selon la revendication 5, dans lequel les deux sections séparables (3a, 3b), suite à l'extension du cathéter (2), sont alignées dans un plan s'étendant suivant une direction de l'extrémité proximale du cathéter.

6. Dispositif médical selon la revendication 5 ou 6, dans lequel les deux sections séparables (3a, 3b) se séparent avec un angle de séparation incliné opposé.

7. Dispositif médical selon l'une quelconque des revendications 5 à 7, dans lequel les deux sections séparables (3a, 3b) sont une continuation intégrale de l'élément d'extension (3).

8. Dispositif médical selon l'une quelconque des revendications 5 à 7, dans lequel les deux sections séparables (3a, 3b) sont reliées à l'extrémité de mesure (9) de l'élément d'extension (3).

9. Dispositif médical selon l'une quelconque des revendications précédentes, comprenant en outre une unité de détection de force connectée à l'élément d'extension (3) pour la détection d'une force de manoeuvre appliquée à l'élément d'extension (3).

10. Dispositif médical selon l'une quelconque des revendications précédentes, dans lequel l'élément d'extension (3) est agencé en rotation dans le cathéter (2) pour apposition à ladite au moins une commissure.

11. Dispositif médical selon l'une quelconque des revendications précédentes, dans lequel l'extrémité de mesure (9) de l'élément d'extension (3) comprend des moyens d'ancrage (6) pour attacher des ancres à au moins une commissure pour l'implant d'annuloplastie.

12. Dispositif médical selon la revendication 12, dans lequel les ancres comprennent au moins une unité ou bague de guidage.

13. Dispositif médical selon l'une quelconque des revendications précédentes, comprenant en outre un limiteur de cuspide (3d) agencé au niveau de l'élément d'extension (3).

14. Dispositif médical selon la revendication 14, dans lequel le limiteur de cuspide (3d) est auto-expansible d'un mode de distribution à un mode déployé.

15. Dispositif médical selon l'une quelconque des revendications 1 à 4 ou 10 à 15, dans lequel l'élément d'extension (3) est formé comme une boucle continue unique ou d'une seule pièce.

16. Dispositif médical selon la revendication 16, dans lequel l'élément d'extension (3) comprend une boucle continue présentant une portion distale courbée vers l'extérieur dans un sens partant de l'extrémité distale (8) du cathéter (2) .
